# EUROPEAN PATENT APPLICATION

(11) **EP 4 464 350 A1**
(43) Date of publication of application: **20.11.2024**
(21) Application number: 23173321.3
(22) Date of filing: 15.05.2023
(51) Int. Cl.: A61M 11/00, A61M 15/00, A61M 16/00, B05B 1/00

(54) **INHALATION THERAPY DEVICE**

(71) Applicant: PARI Pharma GmbH, 82319 Starnberg (DE)
(72) Inventor: STOECKL, Carolin, 80331 München (DE); HOFFMANN, Tobias, 86938 Schondorf am Ammersee (DE); HEINE, Benjamin, 80687 München (DE); REINHART, Markus, 86919 Utting (DE); DENIS, Thomas, 82319 Starnberg (DE)
(74) Representative: Hoffmann Eitle

(57) **Abstract**

Inhalation therapy device comprising: a housing body (4), a reservoir (2) for holding a liquid in the housing body (4), a membrane (5) disposed in the housing body (4) and having a plurality of apertures (110), wherein the liquid is suppliable to a first side (5.1) of the membrane (5) by gravitational force; an actuator (6) coupled to the membrane (5) for vibrating the membrane (5), whereby the liquid passes through the apertures (110) and an aerosol is generated at a second side (5.2) of the membrane (5) opposite to the first side (5.1); a flow path (7) being defined in the housing body (4) and having a first opening (8) to the outside of the housing body (4) at one end and at least one second opening(-s) (9) to the outside of the housing body (4) at another end, so that a flow is generatable in a first flow direction (A) from the second opening(-s) (9) to the first opening (8) in the flow path (7) upon inhalation of a user at the first opening (8) for entraining and delivering the generated aerosol, and/or in a second flow direction (B) from the first opening (8) to the second opening(-s) (9) in the flow path (7) upon exhalation of a user at the first opening (8); a sensor (47) configured to detect a flow parameter of the flow in the flow path (7), a controller (18) configured to conclude based on the output of the sensor (47) at least on an inhalation phase and/or an exhalation phase of a user and to, during a single treatment time, repeatedly operate the actuator (6) during a period of the inhalation phase and not operate the actuator (6) during a period of the exhalation phase, wherein an aerosol output rate of the inhalation therapy device at the first opening (8) during the single treatment time is, when using a test solution of albuterol 0,1 % (M/V) concentration in 0,9 % sodium chloride as the liquid, at least 0.48 g/min, optionally at least 0.64 g/min and more optionally at least 0.8 g/min at a Median Mass Diameter of aerosol droplets between 4.5pm and 5.0µm, or at least 0.42 g/min, optionally at least 0.54 g/min and more optionally at least 0.70 g/min at a Median Mass Diameter of aerosol droplets between 4.0µm and 4.5pm, or at least 0.29 g/min, optionally at least 0.38 g/min and more optionally at least 0.51 g/min at a Median Mass Diameter of aerosol droplets between 3.5pm and 4.0µm, or at least 0.20 g/min, optionally at least 0.29 g/min and more optionally at least 0.38 g/min at a Median Mass Diameter of aerosol droplets between 3.0µm and 3.5pm, or at least 0.13 g/min, optionally at least 0.19 g/min and more optionally at least 0.26 g/min at a Median Mass Diameter of aerosol droplets between 2.5pm and 3.0µm.

## Description

### Technical Field

The present disclosure relates to an inhalation therapy device for generating and delivering an aerosol to a user.

### Background

Inhalation therapy devices, also known as (inhalation) nebulizers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMD (mass median diameter) of less than Spm such as between 1µm and Spm or between 1µm and 4µm and optional at least 1µm are required to allow deposition in the intended part of the respiratory system.

The aerosols for therapeutic purposes are generated and delivered to a desired location within the user's body, especially its respiratory tract, with the inhalation therapy device. To do so, a fluid, particularly a liquid, i.e., a medicament, a drug, a vaccine, etc., to be aerosolised or nebulised is supplied to a reservoir for holding the fluid or liquid in the inhalation therapy device. For aerosolising or nebulizing the liquid in the reservoir, inhalation therapy devices may, for example, comprise a membrane unit.

Such membrane units comprise a membrane having a plurality of apertures and an actuator directly or indirectly, via a support plate supporting the membrane, coupled to the membrane for vibrating the same.

The fluid or liquid may be in contact with a first side of the membrane via gravitational force. The liquid or fluid (i.e., the medicament) passes through the apertures from the first side of the membrane and an aerosol is generated at a second side of the membrane opposite to the first side of the membrane upon vibrating the membrane.

The aerosolised or nebulised liquid or fluid, i.e., the aerosol, inside the inhalation therapy device is then supplied to the user's respiratory system, within the bounds of an inhalation therapy upon inhalation of the user through the inhalation therapy device. Meaning, upon the application of a suction force by the user's breathing, especially his or her inhalation, through the inhalation therapy device the aerosol is at least partially transported (by suction force) from the interior of the inhalation therapy device to the patent's respiratory tract.

An example for a conventional inhalation therapy device having such a configuration is, for example, derivable from DE 199 53 317 C1. The membrane unit of the disclosed inhalation therapy device comprises a cylindrical liquid reservoir container, which is delimited at one end face by a membrane having the shape of a circular disc. A liquid disposed in the reservoir contacts the side of the membrane facing the reservoir.

DE 199 54 327 C1 further discloses a membrane connected (welded) to a substrate (support plate) and an oscillating generator, for example, a piezo crystal, which surrounds the membrane in a circular manner and is connected (glued) to the substrate, such that the membrane can be caused to oscillate by means of the oscillating generator and an electric drive circuit. Thereby, the liquid applied to the membrane on the first side of the membrane is conveyed through the apertures in the oscillating membrane to the second side of the membrane opposite to said first side of the membrane to be emitted into a chamber as an aerosol. The disclosed inhalation therapy device further embodies a mouthpiece or mask connected to an outlet of the chamber and having an exhalation valve. Upon exhalation of a user, the exhaled flow will, thus, not or only to a limited extend enter the chamber and rather be expelled through the exhalation valve into the environment of the device.

The disclosed device continuously drives the piezo crystal throughout the treatment time (single administration interval), in which substantially the entire liquid in the reservoir is aerosolized by the membrane. During exhalation of a user, the generated aerosol is temporarily stored in the chamber forming an aerosol cloud (bolus), which upon inhalation is inhaled and transported to the respiratory tract for therapy. Therefore, the treatment time can be kept relatively short. One potential drawback is, however, that with an increasing total output rate of the aerosol generator (membrane, piezo crystal, drive circuit, etc.) the amount of aerosol deposited on the walls of the device (rainout), particularly in the chamber, and thereby, loss of administrable aerosol/pharmaceutical increases.

Other conventionally known inhalation therapy devices, drive the piezo crystal only during inhalation and stop driving the piezo crystal during exhalation. Those inhalation therapy devices are often referred to as breath-triggered devices or as breath-actuated devices. It has been found that such inhalation therapy devices often have the issue of a long treatment time (long administration interval), which can be annoying to the user and, thereby, provoke a lack of user adherence, e.g. an interruption or a premature end of therapy before the intended dose of liquid in the reservoir has been nebulised and delivered to the user's respiratory tract for therapy.

Both of the conventional inhalation therapy devices described above are oftentimes operated in such a manner that the user merely inhales through the inhalation therapy device (an inhalation flow is generated through a flow path of the device) and the inhalation therapy device is removed from the user's mouth and/or nose upon exhalation or comprises an exhalation valve in the mouthpiece or mask for expelling the exhaled air. While both enables a resistance-free exhalation of the user, the overall treatment cannot be satisfactorily monitored as no data, such as a flow parameter in a flow path of the device, can be obtained during the exhalation. Additionally, and in case of a continuous operation, generated aerosol gets lost without any therapeutic effect for the user.

Hence, there remains a need for a simple and effective inhalation therapy device that enables a short overall treatment time (single administration interval).

### Summary

In view of the aforesaid, it is an object of the present disclosure to establish an inhalation therapy device that increases or at least maintains the overall quality of drug delivery while keeping the overall treatment time as short as possible.

This object is solved by means of an inhalation therapy device according to independent claim 1. Distinct embodiments are derivable from the dependent claims and the following description.

According to a first aspect, an inhalation therapy device is suggested. The inhalation therapy device comprises a housing body and a reservoir for holding a liquid, the reservoir being accommodated or formed in the housing body. The reservoir may be an integrated portion of the housing body. In one example, the reservoir and the housing body may be integrated as one piece, e.g. in an injection moulding process. In another example, the reservoir and the housing body may be separately manufactured and integrated by being fixed, such as non-detachably fixed, to each other. Alternatively, the reservoir may be an ampoule exchangeably inserted into and opened in the housing body. The usual amount of liquid to be contained in the reservoir is 1ml to 12ml or 1ml to 8 ml, however, 1ml to 6ml are also possible.

The inhalation therapy device further comprises a membrane having a plurality of apertures (through holes). The membrane may be a circular disk having an active area in its center, wherein the apertures are formed in the active area. The membrane may be manufactured from metal, for example from stainless steel, or other suitable materials. The membrane is disposed or accommodated in the housing body. The liquid in the reservoir is suppliable to a first side of the membrane by gravitational force. The reservoir may for this purpose have an outlet opening surrounded by a sealing lip abutting the surface of the membrane on the first side and surrounding the active area.

The inhalation therapy device further comprises an actuator coupled to the membrane for vibrating the membrane. The actuator may be a piezoelectric element, such as a piezoelectric ring. The actuator may be directly coupled or attached to the membrane, that is either to the first side of the membrane or to a second side of the membrane opposite to the first side. Alternatively, the membrane may be coupled or attached to a support plate, such as an annular support plate and the actuator may be directly coupled or attached to the support plate. Upon activating or operating the actuator, the membrane is vibrated. Upon vibration of the membrane, the liquid passes through said apertures and an aerosol is generated at the second side of the membrane opposite to the first side. The membrane and the actuator may constitute components of a membrane unit (nebulizer head).

The inhalation therapy device further comprises or defines a flow path. More particular, the flow path is defined in or by the housing body and comprises a first opening to the outside (or the environment) of the housing body (or the inhalation therapy device) at one end of the flow path and at least one second opening to the outside (or the environment) of the housing body (or the inhalation therapy device) at another, opposite end. Accordingly, a flow can be generated in a first flow direction (inhalation flow direction) from the second opening(-s) to the first opening in the flow path upon inhalation of a user at the first opening for entraining and delivering the generated aerosol. To put it differently, upon inhalation of a user at the first opening, a suction force is generated at the first opening, whereby a negative pressure is generated in the flow path and air is sucked in through the second opening(-s) entering the flow path. Thereby, an airflow is generated which entrains the generated aerosol and delivers it via the first opening to the user. Similarly, a flow can be generated in a second flow direction (exhalation flow direction) from the first opening to the second opening(-s) in the flow path upon exhalation of a user at the first opening. Upon exhalation of a user at the first opening, exhaled air is "blown" via the first opening into the flow path, whereby a positive pressure is generated in the flow path, passes through the flow path and exits the flow path at least via the second opening(-s).

In order to enable entraining the generated aerosol for delivery to the user, the membrane or the membrane unit may be disposed in the flow path between said first opening and said second opening(-s). This configuration is not limited to an inline arrangement of the membrane or membrane unit, wherein the membrane unit is disposed in the air flow between the first and second openings in the flow path itself. Thus, it is possible to arrange the membrane or membrane unit in such a manner directly/inline in the flow path that it can at least be partially surrounded by the flow in the flow path. In other words, an envelope or sheath flow is generated about the circumference or part of the circumference of the membrane (see later). Alternatively, the membrane or membrane unit can also be arranged in a separate channel being in fluid communication with the flow path such that the generated aerosol can equally be emitted into or transported to the flow path. An example for such a configuration is a T-connection of the channel to the flow path.

The inhalation therapy device further comprises a sensor configured to detect or sense a flow or a flow parameter of the flow in the flow path. In this context, it is not required that the sensor itself is disposed in the flow path. Rather, it is sufficient that some kind of fluid communication is realized between the flow path and the sensor. For this purpose, a sensor or measurement port may be located within the flow path or in a wall of the flow path and be fluidly communicated with the sensor, e.g. via a tube or hose. Various kinds of flow parameters to be detected are conceivable in this regard, such as pressure (absolute pressure, static pressure, total pressure, dynamic pressure or differential pressure), volume flow rate, mass flow rate, acoustic noise, vortex shedding, etc..

Moreover, the inhalation therapy device comprises a controller. The controller may be accommodated in the housing body. The controller is configured to conclude based on the output of the sensor at least on an inhalation phase (inhalation) and an exhalation phase (exhalation) of a user. In other words, the controller may via the output of the sensor conclude on the breathing cycle of the user, such as start of inhalation, end of inhalation, start of exhalation and end of exhalation, breathing frequency, inhalation/exhalation ratio (inhalation/exhalation ratio is the ratio of the amount of time spent inhaling compared to the amount of time spent exhaling), flow rate (current, maximum, mean, minimum), inhaled volume (current, maximum, mean, minimum), exhaled volume (when exhaled through device), minute volume, derivatives of volume, and the like. In an example, the controller may interpret e.g. pressure signals in different ways and thus calculate e.g. the inhaled/exhaled volume, which in turn may be used for the later described triggering (actuation/operation of the membrane).

Furthermore, the inhalation therapy device is intended to administer a medicament or drug contained in the liquid in one session. To put it differently, the inhalation therapy device, during a single treatment (single administration interval or single treatment time), transfers substantially all of the liquid contained in the reservoir into an aerosol and delivers the aerosol to the user. The treatment time may be the time required to deliver a defined amount of drug to (the lungs of) a user. A single treatment may start after the inhalation therapy device is switched ON and the actuator is subsequently operated/activated/actuated for the first time, such as when the sensor for the first time senses an inhalation flow, and end when no further liquid is contained in the reservoir or when a residual of liquid remains in the reservoir which can no longer be aerosolized, e.g. is trapped in the reservoir. In other words, the treatment time may be defined as the time period between the first actuation/operation of the actuator and the last actuation/operation of the actuator or the indication of the inhalation therapy device that substantially all liquid within the reservoir has been nebulized or aerosolized (end of dose). In regard of the latter, it is to be emphasized that also a small amount of liquid may remain in the reservoir, e.g. being trapped (reservoir residual volume). The amount of remaining liquid after complete nebulization should, however, be less than 1.2ml, less than 0.7ml or less than 0.5ml.

According to the present disclosure, the controller is configured to repeatedly operate and not operate the actuator during a single treatment (administration interval or treatment time). In other words, the actuator is repeatedly activated and deactivated. In the context, operating the actuator is to be understood as controlling the actuator so as to vibrate the membrane and generate aerosol, whereas not operating the actuator is to be understood as controlling the actuator so as to not vibrate the membrane and not generate aerosol. In particular, during a period of inhalation (at least a time period during inhalation/an inhalation phase), the controller is configured to operate/activate the actuator, whereby the membrane is vibrated and will generate the aerosol. During a subsequent period of exhalation (at least a time period during exhalation/an exhalation phase), the controller is configured to not operate/activate the actuator so that the membrane is not vibrated and no aerosol is generated.

According to the first aspect, an aerosol output rate (AOR) in g/min of aerosol at the first opening during the single treatment time is
a) at least 0.48 g/min, optionally at least 0.64 g/min and more optionally at least 0.80 g/min at a Median Mass Diameter of aerosol droplets between 4.5pm and 5.0pm, or
b) at least 0.42 g/min, optionally at least 0.54 g/min and more optionally at least 0.70 g/min at a Median Mass Diameter of aerosol droplets between 4.0µm and 4.5µm, or
c) at least 0.29 g/min, optionally at least 0.38 g/min and more optionally at least 0.51 g/min at a Median Mass Diameter of aerosol droplets between 3.5pm and 4.0pm, or
d) at least 0.20 g/min, optionally at least 0.29 g/min and more optionally at least 0.38 g/min at a Median Mass Diameter of aerosol droplets between 3.0µm and 3.5µm, or
e) at least 0.13 g/min, optionally at least 0.19 g/min and more optionally at least 0.26 g/min at a Median Mass Diameter of aerosol droplets between 2.5pm and 3.0µm.

The aerosol output rate is defined as mass of aerosol emitted at the first opening by the inhalation therapy device per unit of time. The AOR is, hence, indicative of the aerosol amount per time the user receives.

DIN EN ISO 27427 (version February 2020, particularly Annex C1) inter alia defines how to measure the aerosol output rate of an inhalation therapy device. For the measurement, an albuterol 0,1 % (M/V) concentration in 0,9 % sodium chloride solution mentioned in DIN EN ISO 27427 is used as test solution. Silicon tubes were used as dismountable connectors, e.g. for connection to the first opening. A PALL filter was used as collection filter. Data M AZS-M13-V7 or Copley BRS-300 were used for the measurement. The test conditions were set as follows:

| **Shape** | **Tidal-volume [ml]** | **breathing frequency [1/min]** | **I/E ratio [-]** | **max. inhalation flow [L/min]** | **max. exhalation flow [L/min]** |
|---|---|---|---|---|---|
| Sinus | 500 | 15,0 | 1,00 | 23,56 | 23,56 |

| **Breath duration [s]** | **Duration inhalation [s]** | **Duration exhalation [s]** | **minute volume [L]** |
|---|---|---|---|
| 4,00 | 2,00 | 2,00 | 7,5 |

The mass median diameter (MMD) is determined by laser diffraction as also disclosed in "Effects of flow pattern, device and formulation on particle size distribution of nebulized aerosol", Junhua Hu, et al., International Journal of Pharmaceutics Volume 560, 5 April 2019, Pages 35-46.

The mass median aerodynamic diameter is determined by cascade impaction as described by USP<1601> or ISO 27427. Ideally, MMAD and MMD are identical for spherical particles with the density of 1.0 g/ml as is the case for most aqueous aerosols produced by nebulizers. In an aspect, the geometric standard deviation (GSD) is lower than 2.0, preferably between 1.6 to 1.8, and most preferably smaller than 1.5.

As laser diffraction measurements are much easier to conduct than impactor experiments, MMD data are shown here. Droplet Size Distribution by Laser Diffraction (LD) was performed by laser diffraction by following test method:
A laser diffractometer Helos BF or BR of Sympatec GmbH including lens R3 was used for this purpose. The inhalation therapy device was located in a chamber through which conditioned air was introduced to transport the aerosol into the laser beam. The used instruments were an automatic conditioning unit (ACU), an analytical balance, XS603S DR of Mettler Toledo, an air conditioning, SV462, Euroline plus of Walter Roller GmbH and a multipette of Eppendorf.

The measurement conditions were as follows:

| | |
|---|---|
| Nebulization time: | 2 minutes or over whole treatment time with determination of optical concentration and MMD [µm] every 10 sec. |
| Ambient temperature: | 23.0 ± 2.0 °C |
| Ambient humidity: | 50.0 ± 5.0 % r.h. |
| Inspiratory Flow: | 20.0 ± 1 L/min: |
| Exhaust air flow: | 90 ± 5 L/min |
| Fill volume: | 4 mL |

The test solution was filled in the reservoir by the pipette. The inhalation therapy device was weighed after filling with the test solution and connected to the laser diffraction test system. Measurements were conducted for 2 minutes or over the whole treatment time with determinations of optical concentration (opt.conc.) and MMD every 10 seconds. The inhalation therapy device was weighed again after completion of nebulization process. Residue in the inhalation therapy device represents the weight of remaining solution in the reservoir and condensate in the flow path.

The treatment time for a desired dose is determined by the aerosolization rate and the efficiency of the device. The aerosolization rate can be determined by the weight loss of the nebulizer during continuous nebulization for a period of time. The weight loss during constant nebulization of the test solution divided through the nebulization time is called total output rate (TOR) and is specified in g/min. Hence, the TOR is indicative for the rate of aerosol generation during constant nebulization. In the above-described laser diffraction method, the total output rate (TOR) of the membrane unit may be calculated upon weight loss through nebulization time.

TOR and AOR are linked through the efficiency of the nebulizer, the proportion of aerosolized liquid delivered to the patient. Particularly, the aerosol output rate is inter alia governed by the total output rate (TOR) of the membrane unit and potential losses of aerosol, e.g. in the flow path of the inhalation therapy device. The higher the TOR of the membrane unit, the higher the aerosol output rate. The lower the losses, the higher the aerosol output rate. From a simplified point of view, the aerosol output rate is the TOR multiplied by the delivered dose (delivered dose (expressed as µg salbutamol) is the amount of drug that is deposited on an inhalation filter and determined by high-performance liquid chromatography. The delivered dose represents the dose available to be inhaled by a user. As an example, a membrane unit constantly producing an aerosol with a MMD of 4.4um at a rate of 0.8g/min (TOR) of which 45% are delivered to the patient results in a corresponding AOR of 0.8g/min (TOR) x 45% = 0.36 g/min. If the membrane unit, in the example above, would be operated only partly during inhalation (breath actuated, e.g. 40% of the entire breathing cycle (inhalation manoeuvre)) 80% of the aerosolized liquid would be delivered to the user. The resulting AOR is then 0.8g/min (TOR) x 0,4 (on time/total time) x 0,8 = 0.26 g/min.

All parameters, including those relating to AOR, TOR, MMAD, MMD, etc., in this disclosure are to be taken at ambient pressure in the reservoir and, hence, without negative pressure in the reservoir.

In an embodiment, the total output rate (TOR) of the membrane unit may be
a) at least 1.5 g/min, optionally at least 2.0 g/min and more optionally at least 2.5 g/min at a Median Mass Diameter of aerosol droplets between 4.5pm and 5.0µm, or
b) at least 1.3 g/min, optionally at least 1.7 g/min and more optionally at least 2.2 g/min at a Median Mass Diameter of aerosol droplets between 4.0µm and 4.5pm, or
c) at least 0.9 g/min, optionally at least 1.2 g/min and more optionally at least 1.6 g/min at a Median Mass Diameter of aerosol droplets between 3.5pm and 4.0µm, or
d) at least 0.6 g/min, optionally at least 0.9 g/min and more optionally at least 1.2 g/min at a Median Mass Diameter of aerosol droplets between 3.0µm and 3.5pm, or
e) at least 0.4 g/min, optionally at least 0.6 g/min and more optionally at least 0.8 g/min at a Median Mass Diameter of aerosol droplets between 2.5pm and 3.0µm.

The combination of triggering the operation/activation of the actuator and thereby the aerosol generation and a minimum aerosol output rate as defined above enable a relatively short treatment time as well as reduced losses caused for example by deposition of aerosol on inner surfaces of the flow path, or increased droplet sizes due to coalescence of droplets and others in the housing body. Considering a 0.5ml or 1ml filling volume of liquid in the reservoir, one may realize treatment with 10 breathing cycles (10 exhalation phases and 10 inhalation phases). Overall, the inhalation therapy device is therefore more convenient to use and provides improved quality of aerosol generation.

It is to be noted that all following aspects may also be separately embodied in an inhalation therapy device not realizing an aerosol output rate in g/min of aerosol emitted by the inhalation therapy device at the first opening during the single treatment defined in claim 1.

According to a second aspect, the controller is configured to, within each breathing cycle including one inhalation phase and one exhalation phase, start operating/activating the actuator within the exhalation phase (exhalation period), particularly at the end of the respective exhalation phase and before the subsequent inhalation phase (inhalation period) starts. This process may also be referred to as "Pre On" process in that the aerosol generation already starts at the end of an exhalation phase. Each in this context, however, refers only to all complete breathing cycles including one exhalation phase (exhalation period) and one inhalation phase (inhalation period). Put differently, the "Pre-On" process will only be activated if a preceding, exhalation was detected. The time period during which the actuator is operated during the exhalation phase may be between 50 ms and 500 ms, optionally 100 ms to 300 ms, most optionally 200 ms, related to a standard sinus-shaped breathing pattern of two seconds of inhalation time and two seconds of exhalation time, respectively, and 500 ml of tidal volume. The length of the "Pre-On" phase is dependent on various factors, e.g. in cases in which the later described storage chamber is realized, of the volume of the storage chamber. The larger the volume of the storage chamber, the longer time periods of the "Pre-On" phases are possible.

Thus, aerosol is already present in a sufficiently large amount at the time the user starts inhaling and is immediately inhaled and transported into the respiratory tract. Thus, the to be administered dose can be delivered to the user very efficiently in short time.

According to a third aspect, the inhalation therapy device further comprises a storage chamber (aerosol storage chamber) formed in the flow path between the membrane or membrane unit and the second opening(-s). The storage chamber may be formed by the flow path itself or by increasing the inner dimensions of the flow path between the membrane or membrane unit at the second opening(-s) to provide for a sufficiently large storage chamber at the same time maintaining a relatively compact housing body.

This aspect is particularly advantageous in combination with the above second aspect. In detail, if the actuator is already activated during the exhalation phase, particularly at the end of exhalation phase, the exhalation flow will tend to wash the generated aerosol out of the aerosol therapy device into the environment and hence it will be lost for therapy. The storage chamber provides for a volume of the flow path between the membrane or membrane unit and the second opening(-s) sufficiently large to temporarily store the aerosol transported by exhalation towards the second opening(-s) and, thus, prevents the aerosol from being expelled from the aerosol therapy device such as from the second opening(-s). The aerosol generated during exhalation, particularly at the end of exhalation, and temporarily stored in the storage chamber is, upon inhalation of the user, again sucked from the storage chamber and delivered to the user via the first opening. As a result losses of aerosol and, hence, e.g. the pharmaceutical, are minimized while the treatment time is shortened.

According to a fourth aspect, the volume of the storage chamber is between 10ml and 100ml, optionally between 20ml and 80ml, more optionally between 25ml and 75ml. The volume is defined between a plane formed by the membrane and the second opening(-s).

It has been proven that these volume ranges provide for a sufficiently large storage chamber to achieve the above effect for a large variety of users and breathing patterns as well as a relatively compact housing body and, therefore inhalation therapy device.

According to a fifth aspect, the sensor is configured to sense the flow parameter at the membrane or between the membrane or membrane unit and the second opening(-s). In this context, either the membrane itself may be used as sensor or the sensor or at least the sensor port is arranged between the membrane or membrane unit and the second opening(-s). In an embodiment in which the above-mentioned storage chamber is provided, the sensor or the sensor port may be located in the storage chamber or a wall of the storage chamber.

Consequently, the sensor or sensor port is located within the housing body in a portion of the flow path which is not susceptible for becoming excessively wet. In particular, it is a position in which the likelihood of aerosol hitting and being deposited on the walls of the flow path is relatively low. For this reason, the reliability and sensibility of the sensor can be improved.

According to a sixth aspect, the membrane is disposed in the flow path, i.e. inline, whereby an envelope or sheath flow about at least part of the circumference of the membrane is formed. For example, the membrane may have an annular outer ring, a concentric circular disc having the above-mentioned active area with the apertures and a plurality of spokes connecting the annular outer ring and the circular disc. Through holes/openings are formed between adjacent spokes in the circumferential direction. These holes/openings may be part of the flow path.

According to the sixth aspect, it can be prevented or at least minimized that generated aerosol hits inner surfaces of the flow path during both inhalation and exhalation. During inhalation, the air flow generated in the flow path will form the envelope or sheath flow about the membrane and entrain the aerosol to be output via the first opening. During exhalation, the air flow generated in the flow path may also entrain aerosol generated during exhalation (at the end of exhalation) when implementing the "Pre-On" process and flow it into the optional storage chamber. Either way, it is successfully prevented that the aerosol hits the inner surfaces of the flow path including the optional storage chamber. Accordingly, deposition of aerosol on inner surfaces in the flow path and the associated losses can be avoided.

Especially towards the end, at the end or at the beginning of either inhalation or exhalation, flow rates are low with an increased risk of impaction of aerosol at opposing walls. If the center axis of the membrane (normal to the plane of the membrane), for example of the active area, is oriented so as to exit the first opening without interfering with inner surfaces of the housing body (flow path), the opposing walls are as far away as possible, reducing deposition of aerosol in the flow path and the associated losses even further.

According to a seventh aspect, the controller is configured to, within each breathing cycle including one inhalation phase and one exhalation phase stop operating/activating the actuator within the inhalation phase (inhalation period) and before the exhalation phase (exhalation period) starts, particularly at the end of the respective inhalation phase and before the exhalation phase starts. This process may also be referred to as "Pre-Off" process in that the aerosol generation is already stopped before the end of the inhalation phase (inhalation period). Each in this context, however, refers only to all complete breathing cycles including one exhalation phase (exhalation period) and one inhalation phase (inhalation period). Put differently, the "Pre-Off" process will only be activated if a preceding, inhalation was detected. The time period during which the actuator is stopped at the end of the inhalation phase may be between 100 ms and 1000 ms, optionally 300 ms to 800 ms, most optionally 600 ms before the exhalation phase starts, related to the standard sinus-shaped breathing pattern of two seconds of inhalation time and two seconds of exhalation time, respectively, and 500 ml of tidal volume.

Stopping the aerosol generation already before inhalation is completed has the advantage, that all the aerosol generated and inhaled will have already reached desired locations in the respiratory tract rather than remaining for example in the trachea and the throat, consequently being exhaled in the next breathing cycle. Thus, exhalation of the aerosol that has not yet reached the desired location and hence does not contribute to therapy in the subsequent exhalation phase can be reduced. Accordingly, a more efficiency pharmaceutical use and higher deposition precision in the respiratory tract can be realized while the treatment time remains reasonably short. Additionally, the flow path, particularly between the second opening(-s) and the membrane, may be kept dry. As the liquid to be nebulized/aerosolized may contain compounds which are detrimental for individuals staying in the environment in which the user inhales even though they serve a therapeutic purpose with respect to the disease of the user, it is known in the art to use exhalation filters so as to avoid those components from being discharged (exhausted) into the environment. One example showing such an exhalation filter is EP 1 868 570 B1. Due to the Pre-Off process such filter may be superfluous, because no or only very little aerosol will be expelled from the device upon exhalation. Further, the aerosol generation and therefore the actuator is active for a shorter time which is beneficial for power consumption, especially if the device is powered by batteries.

According to an eighth aspect, the inhalation therapy device further comprises a mixing chamber formed in the flow path between the membrane and the first opening. The mixing chamber may be the volume into which the aerosol is generated or ejected from the membrane.

Thus, the mixing chamber provides sufficient volume to avoid the aerosol colliding excessively with itself and the inner surfaces (walls) of the flow path and producing associated losses.

According to a ninth aspect, the volume of the mixing chamber is between 5 ml and 50 ml, optionally between 10 ml and 40 ml, more optionally between 12.5 ml and 37.5 ml. The volume is defined between the first opening and a plane formed by the membrane.

Such a volume is perceived as being sufficiently large to avoid the aerosol colliding excessively with itself and the inner surfaces (walls) of the flow path and producing associated losses without leading to a bulky device. In an embodiment also comprising the optional storage chamber, the volume ratio between the storage chamber and the mixing chamber may be at least 1, optionally at least 1.5, more optionally at least 2, i.e. the volume of the storage chamber is equal to or larger than the volume of the mixing chamber.

According to a tenth aspect, the mixing chamber tapers towards the first opening. In other words, the mixing chamber has its largest cross-section at the membrane to accommodate the generated aerosol with minimal collisions with the inner surfaces of the mixing chamber. The cross-sectional area of the mixing chamber may from the membrane gradually and/or continuously taper towards the first opening at which the aerosol is output to the user. Alternatively, the mixing chamber will start with a constant cross-sectional area and only subsequently gradually or continuously taper towards the first opening.

The first opening needs to have a certain (smaller) cross sectional area such that the user may comfortably use the device and put it at the first opening into the mouth. According to this aspect, the aerosol is assisted in reaching the first opening without excessively hitting the inner surfaces of the mixing chamber and causing losses due to deposition of aerosol on inner surfaces of the device defining the flow path.

According to an eleventh aspect, the flow parameter is a pressure, optionally a differential pressure and more optionally a differential pressure between the pressure in the flow path and ambient pressure.

It has been shown that the use of the pressure as flow parameter provides for cost effective and at the same time reliable detection/sensing of inhalation and exhalation and to thereby enable an accurate triggering (activation/deactivation) of the actuator. In case of an absolute pressure, two absolute pressure sensors may be provided, e.g. upstream and downstream of a flow restriction disposed in the flow path. If the restriction is located at an outer wall of the housing body (for example being embodied by the second opening or second openings), one of the absolute pressure sensors may be provided to measure the environmental pressure (ambient pressure) while the other of the absolute pressure sensors may be provided to measure the pressure in the flow path of the device. A differential pressure may be used likewise, e.g. one pressure port of the differential pressure sensor upstream and one pressure port of the differential pressure sensor downstream of the flow restriction. If the restriction is located at an outer wall of the housing body (for example being embodied by the second opening or second openings), one pressure port of the differential pressure sensor may be provided to measure the environmental pressure (ambient pressure) while the other pressure port may be provided to measure the pressure inside the flow path. Yet, using a differential pressure sensor taking the pressure in the flow path, optionally between the second opening(-s) and the membrane, and more optionally in the storage chamber, and ambient pressure into account has proven to be most reliable and simple in structure.

According to a twelfth aspect, the flow path is configured to provide a first flow resistance (e.g. maximum absolute values in a range of: 160Pa ± 100Pa; preferred 160Pa ± 60Pa; more preferred 160Pa ± 30Pa; measured at a peak flow of a sinus-shaped breathing pattern of two seconds of inhalation time and two seconds of exhalation time, respectively and 500ml of tidal volume) upon inhalation of a user and a second flow resistance (e.g. maximum absolute values in a range between 120Pa ± 75Pa, preferred 120Pa ± 45Pa, more preferred 120Pa ± 22,5Pa, measured at the peak flow of the sinus-shaped breathing pattern of two seconds of inhalation time, two seconds of exhalation time and 500ml of tidal volume) upon exhalation of the user lower than the first flow resistance. This standard breathing pattern has been used for the definition, but it applies generally for any "symmetrical" breathing pattern, i.e., when the inhalation and exhalation durations are the same and have the same progression. In other words, the second flow resistance upon exhalation (of a user, a pump or a breathing simulator) is at least 25% lower than the first flow resistance upon inhalation (of a user, a pump or a breathing simulator).

This enables, on the one hand, a speedy exhalation against a reduced exhalation resistance. On the other hand, the inhalation resistance is increased compared to the exhalation resistance, such that the inhalation time can be prolonged. In this regard the configuration of an inhalation flow resistance of 160Pa ± 30Pa and said exhalation resistance of a maximum of 80Pa is the preferred range for entraining and delivering the generated aerosol to the user's respiratory tract and still felt convenient for the user. This is because inhaling against such an inhalation flow resistance sufficiently prolongs the inhalation, while not overwhelming the user, and an exhalation resistance of 80Pa or less ensures that the user is not confronted with a too strong resistance upon exhalation, such that exhaling through the device does not lead to a feeling that strongly deviates from a natural breathing behaviour. Increasing the inhalation resistance and decreasing the exhalation resistance at the same time, may offer four main advantages:
i) The maximum flow rate during inhalation is limited due to the increased breathing resistance leading to higher drug deposition in the lungs.
ii)The duration of the inhalation is prolonged leading to a longer window where aerosol can be produced and ultimately to a shorter treatment time.
iii) While the inhalation is prolonged due to an increased breathing resistance, exhalation is shortened due to a decreased breathing resistance. This reduces the breathing effort as the patient is enabled to maintain breathing frequency and minute volume.
iii) The inhalation pressure signal generally plays a more important role in activating/operating the actuator. Having an increased absolute pressure during inhalation facilitates triggering (activating/operating the actuator) and leads to improved therapy (reliability, duration).

The inhalation resistance can be achieved by the geometric shape of the second opening(-s) being shaped as a fixed geometry passive valve, which allows for a particularly simple, compact, yet efficient inhalation therapy device. If all other cross-sectional areas in the flow path will be larger than that of the second opening(-s), the inhalation resistance will exclusively be governed by the second opening(-s).

The exhalation resistance can also be achieved by the geometric shape of the second opening(-s) being shaped as a fixed geometry passive valve, which allows for a particularly simple, compact, yet efficient inhalation therapy device. For example, the second opening(-s) may be configured such that due to hysteretic effects the resistance in one flow direction (here inhalation) is higher than in the other flow direction (here exhalation) as it can be observed for example in a Tesla valve. In this case, air will substantially (except for some leakage in the device) enter (upon inhalation) and exit (upon exhalation) the flow path via the second opening(-s).

Alternatively, the inhalation therapy device may further comprise a third opening or a plurality of third opening(-s) to the outside of the housing body, wherein the third opening(-s) is (are) provided in the flow path between the first opening and the second opening(-s), optionally between the membrane and the second opening(-s), more optionally in the optional storage chamber. The third opening(-s) in the inhalation therapy device may be provided upstream of the membrane unit of the flow path, when seen along the first flow direction. Accordingly, the third opening(-s) can be arranged at a position that is sufficiently far away from the first opening encountering the user's mouth such that unpleasant feelings from the air being exhausted through the inhalation therapy device can be avoided.

This (these) third opening(-s) is (are each) provided with a check valve, which is configured to substantially (except for some leakage through the valve) restrict a flow through the third opening(-s) in the first flow direction (inhalation) and is configured to enable a flow through the third opening(-s) in the second flow direction (exhalation) from the first opening to the second opening(-s) for enabling at least a partial expel of air through the third opening(-s). In an example, not more than half, or not more than a third, of the air escapes through the third opening(-s) (1/2 or 1/3 of the volume flow rate).

Such an inhalation therapy device is configured to allow an inhalation flow through the second opening(-s), as the "check valve" at the third opening(-s) blocks said opening in the flow path upon inhalation, while, upon exhalation through the inhalation therapy device, the third opening(-s) and its check valve provide for a reduced resistance for expelling the air from the inhalation therapy device. As such, part (see above) of the exhausted air can be expelled through the freely opening check valve at the third opening(-s) and does not have to pass the second opening(-s) and its higher exhausting resistance. Hence, the second flow resistance is reduced relative to the first flow resistance and preferred the second flow resistance is at least 25% lower than the first flow resistance.

According to a thirteenth aspect, the first flow resistance is between 1.25 times and 6 times, optionally 1.5 and 3 times, and more optionally 1.5 times and 2.5 times as large as the second flow resistance.

Such ratios have proven advantageous in limiting the inhalation flow rate, assisting prolongation of the inhalation phase and shortening the exhalation phase, increasing the inhalation flow parameter signal, which results in an overall higher therapy success, without cause discomfort, such as breathlessness, by the user.

According to a fourteenth aspect, the apertures penetrate the membrane in an extension direction from the first side to the second side, each aperture having along its extension direction at the second side a nozzle portion (smallest diameter portion), wherein the length of the nozzle portion is between 5 µm and 16 pm, optionally 5 µm and 14 pm, more optionally Spm and 12um. In this regard, the nozzle portion may, depending on its manufacturing method, be funnel-shaped or tapered with its smallest cross-sectional area (diameter) perpendicular to the extension direction of the apertures at the second side.

The present invention is based on the finding that the length of the nozzle portion of the apertures formed in the vibratable membrane has a significant influence on the total output rate (TOR) and, hence, the aerosol output rate (AOR). In particular, the length of the nozzle portion is directly proportional to the TOR, wherein the shorter the nozzle portion, the higher the TOR and vice versa. In contrast, the diameter and the length of the portions upstream of the nozzle portion within the aperture have only a small influence on the TOR, if the nozzle portion is sufficiently short and small in diameter as compared to the upstream portion of the aperture. In this context, it is preferred that the diameter of the nozzle portion at the second side of the membrane is between 1 µm and 5 pm, optionally between 1 µm and 4 pm, more optionally between 2 µm and 3 µm. On the other hand, the diameter distribution of the nozzle portion as well has an influence on the geometric standard deviation (GSD) of the droplet size distribution. Low GSDs characterize a narrow droplet size distribution (homogeneously sized droplets), which is advantageous for targeting aerosol to the respiratory system. The particle size (below Spm) has a GSD lower than 2.0, preferably between 1.6 to 1.8, and most preferably smaller than 1.5.

The length of the nozzle portion may be defined as that portion starting from the second side towards the first side up to the next laser drilling stage (see below).

In addition, it is preferred that the total length of an aperture in the extension direction and, hence the thickness of the membrane, is at least 40 µm, preferably at least 70 µm and most preferred at least 100 and at most 110 µm.

According to a fifteenth aspect, the apertures are laser drilled formed in at least two stages, one stage forming the nozzle portion and the remaining stage(-s) forming the remainder of the respective apertures.

The vibratable membrane may comprise a single (one single) layer of unitary material. A single layer of unitary material has no distinct borders, boundary surfaces or confining layers or the like in a polished cut image of the single layer. In an embodiment, the vibratable membrane may consist of the single layer.

Instead of realizing the apertures by two or more laser drilling stages completely penetrating the single layer of unitary material as a through hole, a plurality of recesses may be formed in the single layer, e.g. via laser milling or etching. The recesses each have an opening at the fluid side (first side) of the vibratable membrane. The recesses each have a bottom opposite to the opening, i.e. at a side facing the aerosol side (second side). The recesses may also be referred to as blind holes introduced from one side of the single layer (from the fluid side (first side) of the vibratable membrane). In one aspect, at least two recesses are provided. In another aspect, at least three recesses are provided. Further, the number of recesses is not limited but less than 3,000, less than 1,000 or less than 500 may be preferred. Alternatively, less than 50 or less than 20 recesses may be provided. A relatively large number of smaller recesses, such as between 500 and 3,000 or 500 and 1,000, may be beneficial to achieve a high stability of the vibratable membrane. The more recesses are provided in the vibratable membrane, the higher the remaining reinforcing portions between the recesses providing for the full thickness of the single material layer and, hence, stability. To the contrary, a smaller number of larger recesses, such as between 2 and 50 or 2 and 20, may be beneficial to achieve a larger effective area for nebulization with less dead areas. In this context, "dead areas" are those areas of the vibratable membrane having no penetrating apertures and, hence, do not participate in the nebulization process. To put it differently, in these areas no fluid may pass through the vibratable membrane for being nebulized at the fluid side. Additionally, the manufacturing process may be simplified when using a smaller number of recesses. The area of the recesses relative to the total area of the vibratable membrane, in one example, does not exceed 50%.

Further, a plurality of apertures is formed in the bottom of each of the recesses. The plurality of apertures may each have an entrance opening formed in the bottom of the respective recess and an exit opening at the aerosol side (second side) of the vibratable membrane. According to an aspect, there are no additional apertures formed in the vibratable membrane other than those formed in the bottom of the recesses. According to another aspect, there are at least no additional apertures formed in the single layer between recesses and being blind holes. Thus, the fluid on the fluid side of the vibratable membrane passes, upon vibration of the vibratable membrane, through the recesses and apertures and is nebulized while exiting the apertures via the exit openings at the aerosol side of the vibratable membrane. Thus, an aerosol is formed at the aerosol side of the vibratable membrane for inhalation by a user. In this aspect, the entrance opening of the apertures can be relatively small so that the density of the apertures may be increased as compared to the density of the three-stage laser-drilled apertures, thus enabling to increase the TOR.

Even further, the TOR and MMD, and as a result the aerosol output rate, are strongly dependent on the geometry of the nozzle portion, i.e. of the aperture geometry (particularly length and diameter). Due to the formation of the recesses, a small and relatively even or constant thickness of the bottom of the recesses is achieved. Accordingly, the length of the apertures is very well defined. In addition, the formation of relatively short apertures is much more precise regarding diameter at an entrance side (corresponding to the fluid side) and an exit side (corresponding to the aerosol side). As a result, a much more consistent geometry of the apertures relative to each other may be achieved, reducing the GSD. Hence, the characteristics of the aerosol (nebulized fluid) are more consistent when comparing different aperture plates obtained from the same manufacturing process.

In one embodiment, the recesses are laser milled and the apertures are laser drilled. The use of a laser milling process for forming the recesses is also beneficial from the viewpoint of achieving a relatively even and constant remaining material thickness in the area of the bottom of the recesses. Thus, an even more precise formation of the apertures regarding diameter and length may be achieved with benefits relating to reproducibility of constant aerosol characteristics. In a specific example, an ultra-short-pulse laser is used for laser milling. An ultra-short-pulse laser is defined as a laser using laser pulses of less than 10 picoseconds. An ultra-short-pulse laser has the benefit of being more precise and even further improving the benefits relating to reproducibility of constant aerosol characteristics described above. The apertures may still be formed by using a short-pulse laser (e.g. a nanosecond laser) or by using an ultra-short-pulse laser. A short-pulse laser is defined as a laser using laser pulses of greater than 10 picoseconds and smaller than 500 nanoseconds.

In addition, it has been found that the TOR may be further increased when increasing the number of apertures provided in the membrane be it by laser drilling in two or more stages as through holes completely penetrating the membrane from the first side to the second side or by first providing recesses and forming the apertures only in the bottom of the recesses. This may either be achieved by increasing the active perforated surface (active area) of the membrane and maintaining the distance of the apertures relative to each other at the same level, or by means of reducing the distance of the through holes relative to each other and maintaining the active area of the membrane. In addition, these measures may as well be combined. From this perspective, it is advantageous that the membrane comprises between 4,000 and 12,000 of the apertures, optionally between 5,000 and 11,000 of the apertures and more optionally between 5,000 and 10,000 of the apertures.

Moreover, the density of the apertures may be between 140 per mm² and 3800 per mm², optionally between 170 per mm² and 3800 per mm², more optionally between 170 per mm² and 2300 per mm². The active area of the membrane may be between 3 mm² and 28 mm², optionally between 7 mm² and 20 mm², more optionally between 10 mm² and 15 mm². The distance between the two adjacent apertures, that is the center axes of adjacent apertures is between 15 µm and 100 pm, optionally between 30 µm and 80 pm, more optionally between 40 µm and 60 µm.

### Brief Description of the Drawings

Hereinafter, non-limiting examples of the disclosure are explained with reference to the drawings, in which:
Figure 1 shows an inhalation therapy device according to the present disclosure in an isometric view.
Figure 2 shows a side view of the inhalation therapy device of figure 1 placed on a horizontal surface.
Figure 3 shows a longitudinal cross section of the inhalation therapy device in figure 1.
Figure 4 shows an explosive view of the inhalation therapy device of figure 1.
Figure 5 shows a side view of an inhalation therapy device in a use position.

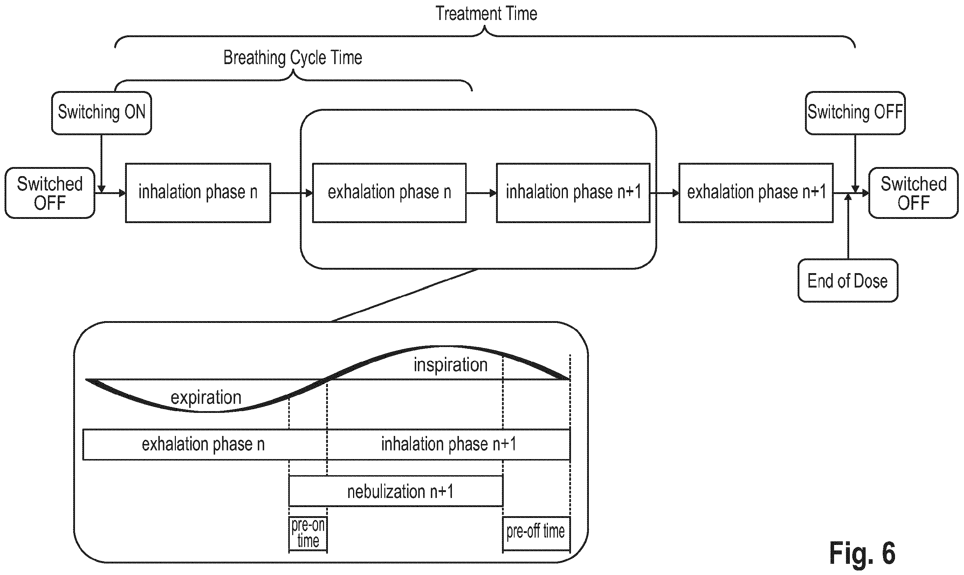

Figure 6 schematically shows the control of an inhalation therapy device of figures 1 to 5.
Figure 7 shows a partial cross section of the active area of the vibratable membrane according to an embodiment.
Figure 8 shows a partial cross section of the active area of the vibratable membrane according to another embodiment.

### Detailed Description

An inhalation therapy device of the present disclosure will now be described with reference to the accompanying drawings.

It is to be understood that such inhalation therapy devices 1 are oftentimes also called "aerosol delivery devices" or "nebulizers", for example for a therapeutic use in/via a user's respiratory system.

Further, the term "user", which has been used throughout the disclosure may be a patient.

In case that specific angles are defined in the present disclosure, positive angles are always taken between respective legs in a counterclockwise direction.

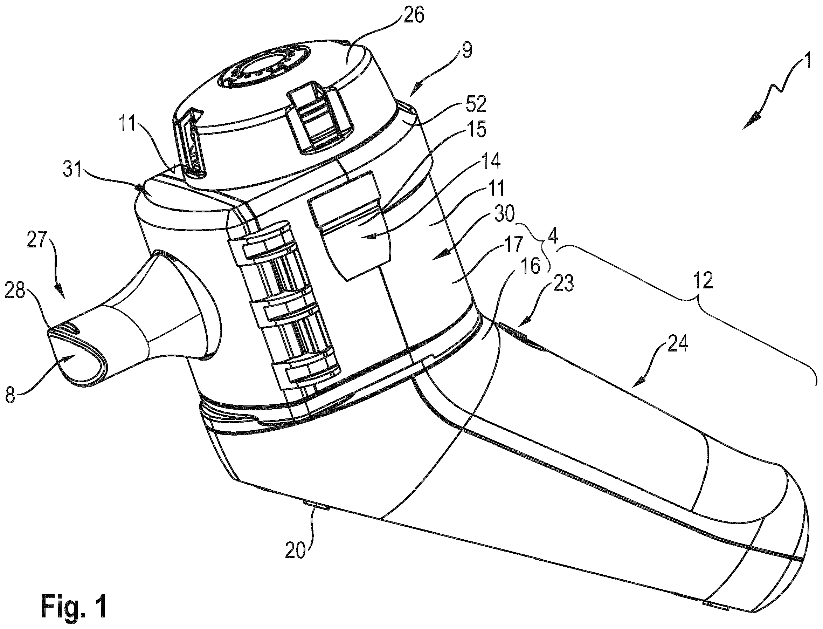

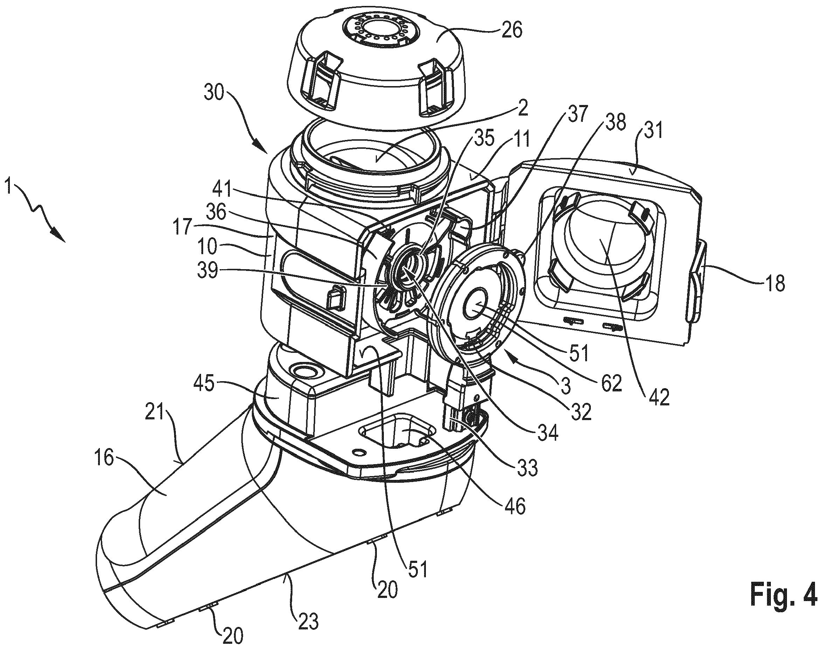

Fig. 1 shows an exemplary isometric view of the inhalation therapy device 1. The inhalation therapy device comprises a housing body 4. The housing body 4 may be split into two main components 16, 17 as best visible from figure 4.

In particular, the housing body 4 comprises a controller housing (which may also be referred to as holding portion or handle portion) 16 and a nebulizer housing 17 (which may also be referred to as aerosolization portion). Yet, the present disclosure is not limited to such a "split" configuration: it may also be possible to form the controller housing 16 and the nebulizer housing 17 integrally.

The controller housing 16, in the embodiment, also functions as or comprises a handle portion 12 during therapy in that the user grips the handle portion and thereby holds the inhalation therapy device 1.

The controller housing 16 further comprises a support surface 19 at its lower side, optionally in the handle portion 12, for placing the inhalation therapy device 1 on a horizontal surface such as a table. The support surface 19 may be defined by a plurality of feet 20 disposed on the bottom surface/lower surface of the controller housing 16. To put it differently, the housing body 4, particularly the controller housing 16, comprises the feet 20 and the support surface 19 is a level surface formed by the respective stand surfaces of the feet 20.

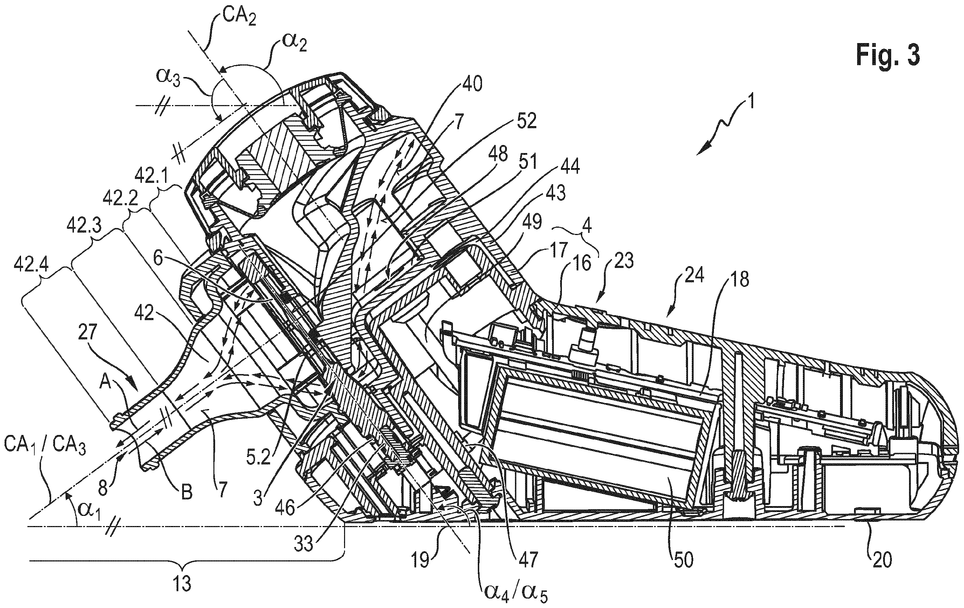

The nebulizer housing 17 has or defines a reservoir 2 for holding a liquid to be aerosolized (see figure 3). The reservoir 2 has a filling opening 25 for receiving the liquid and a lid 26 for closing the filling opening 25 (see figure 4). An angle α₂ between the support surface 19 and a center axis CA₂ of the filling opening 25 is, in side view (longitudinal cross sectional view), an obtuse angle of more than 90°±5°, optionally between 100° and 160° and optionally between 110° and 150°. An angle α₃ between the center axis CA₂ of the filling opening 25 and the center axis CA₁ of the mouthpiece is, in side view (longitudinal cross sectional view), between 70° and 110° and optionally 80° and 100° and more optionally 90° ±5°.

The nebulizer housing 17 further defines a flow path 7 having a first opening 8 to the outside of the housing body 4, particularly the nebulizer housing 17, at one end and at least one second opening 9 to the outside of the housing body 4, particularly the nebulizer housing 17, at another end, so that a flow is generatable in a first flow direction A from the second opening(-s) 9 to the first opening 8 in the flow path 7 upon inhalation of a user at the first opening 8 for entraining and delivering the generated aerosol, and in a second flow direction B from the first opening 8 to the second opening(-s) 9 in the flow path 7 upon exhalation of a user into the first opening 8.

In this connection, the above-mentioned flow in the first flow direction A from the second opening(-s) 9 to the first opening 8 may also be understood as an "inhalation flow". Since the exhalation of the user shall also be performed through the flow path 7 of the inhalation therapy device 1, a second flow direction B from the first opening 8 to the second opening(-s) 9 in the flow path 7 upon exhalation of the user at the first opening 8 is understood as an "exhalation flow".

The first opening 8 is in the present example part of a mouthpiece 27 and defined by an outer edge 28 of the mouthpiece 27. The mouthpiece 27 is configured for being put into the mouth of a user for inhaling the generated aerosol.

The mouthpiece 27 or particularly its first opening 8 defines a center axis CA₁. The center axis CA₁ is non-parallel to the support surface 19. The center axis CA₁ of the first opening 8 intersects the virtual extension 13 of the support surface 19 on a mouthpiece 27 side with respect to the support surface 19 and an angle α₁ between the virtual extension of the support surface 19 and the center axis CA₁ of the first opening 8 is, in side view (longitudinal cross sectional view) (figure 3), an acute angle of less than 75°, optionally between 10° and 70°, optionally between 15° and 60°, optionally between 20° and 50°.

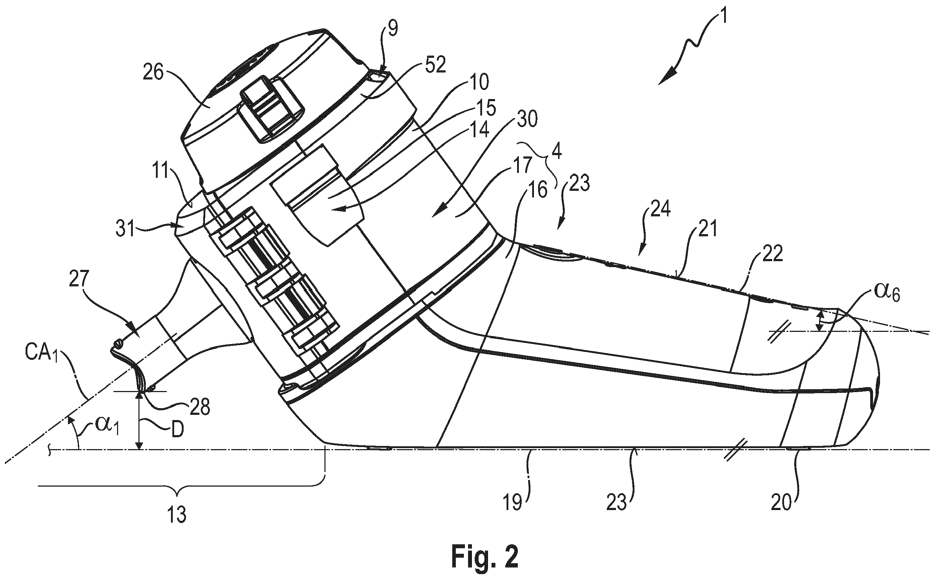

Accordingly, the outer edge 28 of the mouthpiece 27 is oriented in a direction towards the support surface 19. Yet, as will be apparent from figures 2 and 3, the outer edge 28 does not interfere with the virtual extension 13 of the support surface 19 and, hence, if placed on a horizontal surface such as a table, does not come into contact therewith. The minimum distance D between the virtual extension 13 of the support surface 19 and the outer edge 28 of the mouthpiece 27 in a direction perpendicular to the virtual extension 13 of the support surface 19 is more than 3 mm and optionally more than 4 mm and less than 15 mm and optionally less than 12 mm.

Furthermore, a mixing chamber 42 is formed between the first opening 8 and the membrane unit 3, particularly the membrane 5, i.e. downstream of the membrane unit 3 in the first flow direction A. The mixing chamber 42 is a volume defined between the first opening 8 and the membrane unit 3. Considering figure 3, the mixing chamber 42 as a first portion 42.1 with a constant cross-sectional area adjacent the membrane unit 3. In a direction towards the first opening 8, the mixing chamber 42 then abruptly decreases in cross-sectional area being substantially constant in cross-sectional area in a second portion 42.2. In a third portion, continuously tapering portion, 42.3, the mixing chamber 42 continuously tapers towards the first opening 8. The mixing chamber 42 are downstream of the third portion 42.3 a fourth portion 42.4 again with constant cross-sectional area ending in the first opening 8. Yet, it is also conceivable and may even be preferred that the portion 42.2 is omitted.

Moreover, the nebulizer housing 17 comprises a circumferential wall 10, a top wall 11 also comprising the filling opening 25 and a bottom wall 51. A transition between the top wall 11 and the circumferential wall 10 may be chamfered 52. The mouthpiece 27 is connected to the circumferential wall 10.

The second opening(-s) 9 is according to an example located adjacent the top wall 11 and in the present embodiment in the chamfer 52. Accordingly, second opening(-s) 9 are positioned most distant from the handle portion 12 so that the risk of blocking the second opening(-s) 9 by the hand or finger of a user can be minimized. The second opening(-s) 9 serve(-s) as the sole opening(-s) to the flow path 7 via which air may enter the flow path 7 upon inhalation of a user. In addition, the second opening(-s) 9 provide for the smallest cross-sectional area throughout the flow path 7 so that the second opening(-s) 9 exclusively govern the first flow resistance, i.e. inhalation flow resistance.

The nebulizer housing 17 may further comprise a third opening 14 or a plurality of third openings 14, which may also be referred to as exhalation opening(-s). The third opening(-s) 14 may be provided in the circumferential wall 10. The third opening(-s) 14 is (are each) covered by a check valve 15, particularly a flap valve (see figures 1 and 2) covering the third opening(-s) 14 on the outside of the nebulizer housing 17. The check valve 15 is configured to restrict a flow through the third opening(-s) 14 in the first flow direction A. That is, the check valve 15 prohibits any flow through the third opening(-s) 14 of the flow path 7 upon inhalation. However, the check valve 15 is also configured to enable a flow through the third opening(-s) 14 in the second flow direction B from the first opening 8 to the second opening(-s) 9 for enabling at least a partial expel of air through the third opening(-s) 14 upon exhalation. The third opening(-s) 14 to the outside of the housing body 4/nebulizer housing 17 is (are) provided in the flow path 7 between the first opening 8 and the second opening(-s) 9, particularly between a later described membrane unit 3 and the second opening(-s) 9, i.e. in the present embodiment in a storage chamber 40 (described later). To put it differently the third opening(-s) 14 is (are) arranged upstream of the membrane unit 3 when seen in the first flow direction A.

A storage chamber 40 is formed in the flow path 7 between the second opening(-s) 9 and the membrane unit 3, particularly the membrane 5, i.e. upstream of the membrane unit in the first flow direction A. the storage chamber 40 is a volume defined between the second opening(-s) 9 and if present the third opening(-s) 14 and the membrane unit 3.

The membrane unit 3 is accommodated in the nebulizer housing 17. The membrane unit 3 comprises a membrane 5 and an actuator 6. In the technical field given, such membrane units 3 are also known as "head units" or "heads".

The membrane 5 has a first side 5.1 (liquid side) and a second side 5.2 (aerosol side). The membrane 5 may be circular and may have a circular active area 62 in its center. The active area 62 is provided with a plurality of apertures 110. Further, the active area 62 may be domed towards the second side 5.2.

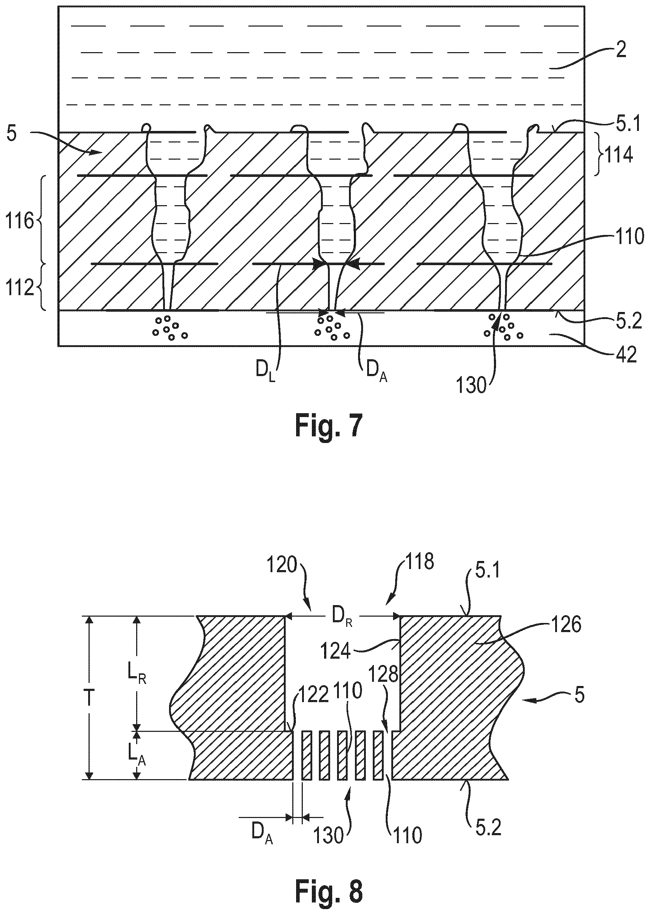

The apertures 110 may be drilled as shown in Figure 7. The apertures 110 penetrate the membrane 61 in the active area 62 from the liquid side 5.1 to the aerosol side 5.2. In use, the liquid solution 26 passes through the apertures 110 from the liquid side 5.1 to the aerosol side 5.2 when the membrane 61 is vibrated for generating the aerosol at the aerosol side 5.2 and emitting it into the mixing chamber 42. This aerosol may then be drawn by inhalation of a user from the mixing chamber 42 via the mouthpiece 27.

Figure 7 shows a cross-section (schematic CT picture) showing three of the apertures 110 of such a vibratable membrane 5. The through holes 110 of this particular embodiment are formed by laser drilling using three stages of different process parameters, respectively. In a first stage, the portion 114 is formed. In a second stage the portion 116 is formed and in a third stage the nozzle portion 112 is formed. In this embodiment, the average length of the nozzle portion 112 is 29.4µm, whereas the second stage portion 116 has an average length of 55.7µm. The first stage portion 114 has an average length of 16.3 µm. As a result, the total length of each aperture 110 is the sum of the length of the first portion 114, the second portion 116 and the nozzle portion 112, that is in this particular example 101.4 µm. Thus, the ratio of the total length of each aperture 110 to the length of a respective one of the nozzle portions 112 is approximately 4.3.

Alternatively, the apertures 110 may be laser milled and laser drilled as shown in Figure 8.

The vibratable membrane 5 in this example, as in the previous example, consists of a single layer 126 of unitary material, e.g. stainless steel. Yet, in other embodiments, the vibratable membrane 5 may comprise further layers attached to the liquid side 5.1 and/or the aerosol side 5.2. Additionally, other biocompatible metals may be used instead of stainless steel.

The exemplary vibratable membrane 5 comprises a plurality of recesses 118. The recesses 118 may be circular or annular.

The recesses 118 have an opening 120 at the liquid side 5.1 of the vibratable membrane 5. The recesses 118 are formed as blind holes having a bottom 122 opposite to the opening 120.

The recesses 118 have a circumferential side wall 124. In case of the circular recess 118, the circumferential side wall 124 corresponds to the sheath of a cylinder. Yet, in the cross-section in Figures 8, portions of the circumferential side wall 124 are opposite to (face) each other.

The recesses 118 may be formed in the single layer 128 by using an ultra-short-pulse laser. In this context, the recesses 118 are preferably made by laser milling, wherein the laser beam and/or the single layer 128 of the vibratable membrane 5 are translated relative to each other whereby material of the single layer 128 is gradually removed.

The smallest dimension D_{R} of the recesses 118 may be 300 µm. However, other dimensions are as well conceivable. For example, a smallest dimension D_{R} of the opening 120 of the recesses 118 facing the liquid side 5.1 may be between 40 µm and 500 µm, between 70 µm and 400 µm or between 90 µm and 300 µm.

In this context, the smallest dimension D_{R} for the circular central recess 118 corresponds to the diameter of the circle. The thickness T of the single layer 128 may be 100 µm.

The depth or length L_{R} of the recesses 118 may be selected between 80% to 95% of the thickness T of the single layer 128. Hence, the depth L_{R} of the recesses 10 may be between 80 µm to 95 µm.

However, the thickness T of the single layer 128 may reside in a range between 50µm to 200µm.

Additionally, the depth L_{R} of the recesses 10 may be selected to be equal to or more than 50% of the thickness T of the single layer 128.

Each recess 118 has a plurality of apertures 110 formed in the bottom 122 and extending from the bottom 122 to the aerosol side 5.2 of the single layer 128.

Each recess 118 may have at least 2, at least 20, or at least 50 or at least 100 of the apertures 110 formed in the bottom 122. At most each recess 118 may have 5,000 or 10,000 of the apertures 110.

Each aperture 110 has an entrance opening 128 at the bottom 122 and an exit opening 130 at the aerosol side 5.2 of the single layer 128. The apertures 110 may be substantially cylindrical or conical with the smaller opening being located at the aerosol side 9 and thus being the exit opening 130. Yet, the geometry is not limited in this regard and other geometries are as well conceivable.

The size of the exit openings 130 of the apertures 110 has a significant impact on the MMD. Depending on the fluid, the MMD is linearly larger than the diameter D_{A} of the exit openings 130 and, thus, directly proportional. Accordingly, the apertures 110 may be circular having a diameter D_{A} (diameter of the exit openings 130) facing the aerosol side 5.2 between 1 µm and 7 pm, preferably between 1.5 µm and 5 pm, and more preferred between 1.5 µm and 4.5 µm and most preferred between 1.5 µm and 3.5 µm. These values apply also to the embodiment in Figure 7.

A length L_{A} (which may also be referred to as height or depth) of the apertures 110 in the single layer 128 may be between 3 µm and 50 µm. In another example, the length L_{A} of the apertures 118 in the single layer 128 may be between 5 µm and 20 µm. In an even further example, the length L_{A} of the apertures 110 in the single layer 128 may be between 10 µm and 20 µm. Other ranges may be 5 to 15pm, 5 to 12µm or 10 to 15µm. In this example of Figure 8, the apertures form the nozzle portion, particularly if formed by only one laser drilling stage. These values apply also to the embodiment in Figure 7.

Even further, the TOR and MMD, and consequently the aerosol output rate, are strongly dependent on the geometry of the nozzle portion 112, i.e. of the aperture 110 geometry (particularly length and diameter).

The apertures 110 may be formed by the same ultra-short-pulse laser used for forming the recesses 118. However, also a short-pulse laser, as used in the prior art, may be used for forming the apertures 110.

In any case, formation of the apertures 110 is preferably performed in one laser drilling stage at a substantially constant fluence of the laser. Only one drilling stage for forming the apertures is preferred to increase preciseness of the geometry of the apertures, i.e. the nozzle portion 112. When using just one laser drilling stage, the diameter at an entrance side (corresponding to the liquid side) and an exit side (corresponding to the aerosol side) will be very well defined with the above described benefits.

In another aspect, the apertures 110 in the bottom of the recesses 118 may also be drilled with two laser drilling stages having a different fluence similar to the embodiment above using the three drilling stages. For example, a first laser drilling stage with a first fluence may be used to form a first part of the aperture in the bottom of the recess and a second laser drilling stage with a second fluence lower than the first fluence may be used to finalize the aperture, i.e., completely penetrate the bottom. In this instance, the nozzle portion may be defined by the second laser drilling stage only. Thus, the bottom may remain thicker (lower depth of the recess) when using more than one laser drilling stage for forming the apertures. This may provide for more mechanical rigidity of the aperture plate.

The method of manufacturing may include as a first step the formation of the recesses 118 from the liquid side 5.1 of the single layer 128.

In a second, subsequent step, the apertures 110 are formed in the bottoms 122 of the recesses 118 from the liquid side 5.1 of the single layer 128.

Subsequently, the vibratable membrane 5 may be domed.

The membrane 5 may be attached to a support. Yet, in the present embodiment, the membrane 5 has an annular portion about its active area 62. The membrane 5 further comprises an annular ring 29 concentric to the membrane 5, particularly its annular portion and active area 62. The annular ring 29 is connected to the membrane 5 via a plurality of spokes 32. Moreover, the membrane 5 has an annular portion about its active area 62. The actuator 6 being directed coupled, that is attached to the annular portion about the active area 62 of the membrane 5.

The actuator 6 is electrically connected to a plug 33 of the membrane unit 3 for electrical connection to the later described controller 18.

As will be apparent particularly from figure 3, the membrane unit 3 is arranged within the nebulizer housing 17 so as to close a supply opening 34 of the reservoir 2 located at the bottom of the reservoir 2. To be more precise, the membrane 5 closes with its first side 5.1 the supply opening 34. To provide a seal, a sealing lip 35 is provided about the circumference of the supply opening 34 (see figure 4). The membrane 5 abuts the sealing lip 35 with its first side so that the active area 62 of the membrane 5 is surrounded by the sealing lip 35. As a consequence, liquid filled into the reservoir 2 will by gravitational force be fed to the supply opening and, hence, to the first side 5.1 of the membrane 5.

To allow replacement and/or cleaning of the membrane unit 3, the membrane unit 3 is interchangeable. In an embodiment, the nebulizer housing 17 comprises a main body 30 and a door 31. The circumferential wall 10 is formed by the main body 30 and to the door 31. The door 31 is hinged to the main body 30. The door 31 may, thus, be moved between an open and a closed position. In the open position (see figure 4) the membrane unit 3 may be inserted into a head mount 36 so that the circumference of the active area 62 at the first side 5.1 of the membrane 5 abuts the sealing lip 35. In order to prevent the membrane unit 3 from being incorrectly mounted, the head mount 36 comprises a key recess 37 into which a key element 38 of the membrane unit 3 is to be inserted during mounting. Upon closing of the door 31, the membrane unit 3 is pressed towards the main body 30, whereby the first side 5.1 of the membrane 5 is pressed against the sealing lip 35. In the closed position, the door 31 is fixed to the main body 30 by using the latch 5.

When placing the membrane unit 3 inside the flow path, a cavity is formed between the membrane unit 3 and the second opening(-s) 9 which would be hard to clean. By configuring the nebulizer housing 17 to comprise the main body 30 and the hinged door 31, the membrane unit 3 may be removed and the cavity is accessible for being cleaned. Yet, providing the latch 18 and configuring the nebulizer housing 17 to comprise the main body 30 and the hinged door 31 is not compulsory. It is also possible to provide a nebulizer housing 17 that can be opened differently by e.g. configuring the main body 30 and the door 31 separately and/or provide some kind of fastening device to fasten them together, such as a magnet, a latch, a press-fitting, a zipper, a press button and the like. Further, it is also possible to provide a nebulizer housing 17 that cannot be opened by a user, operator, hospital staff or the user itself. This is may, for example, be achievable by forming a nebulizer housing 17 in which the membrane unit 3 is arranged in a fixed position and cannot be replaced. In this instance, it may even be conceivable that the whole nebulizer housing 17 is a disposable and will be disposed from time to time.

As will be best apparent from figure 3, an angle α₄ between the support surface 19 and the membrane 5 is, in side view (longitudinal cross sectional view), more than 90° ±5°, optionally between 100° and 160° and more optionally between 110° and 150°.

Taking the aforesaid into account, the nebulizer ho using 17 defines the mouthpiece 27, accommodates the membrane unit 3 and forms the flow path 7 between the first opening 8 and the second opening(-s) 9.

As will be apparent from figure 3, the membrane 5 of the membrane unit 3 is disposed in the flow path 7. In addition, the flow will, during inhalation, enter the housing body 4, particularly the nebulizer housing 17 via the second opening(-s) 9 flowing into the storage chamber 40 formed between the second opening(-s) 9 and the membrane 5 of the membrane unit 3. Moreover, flow path openings 39 (see figure 4) are formed in a wall 41 comprising the head mount 36 so that air may flow from the storage chamber 40 through the flow path openings 39 and the open spaces between the spokes 32 of the membrane 5 into the mixing chamber 42. Accordingly, an envelope flow about at least part of the circumference of the membrane 5 or more particularly in the present embodiment about the circular disc having the active area 62 of the membrane 5 is formed. To put it differently, the membrane unit 3 may be arranged in the flow path 7, such that a sheathing or enveloping flow can pass around the membrane unit 3 inside the flow path 7 during inhalation, and during exhalation, of the user during breathing through the inhalation therapy device 1. In an embodiment, the membrane unit 3 may be arranged substantially (±10° or ±5°) in an axially centred position. In particular, it may be useful that a plane normal of the membrane is as parallel as possible to the mouthpiece axis CA₁). In order to deposit as little aerosol as possible in the device, direct nebulization in the direction of the first opening 8 is desired. For this purpose, a (nearly) perpendicular position of the membrane 5 to the mouthpiece axis CA₁ is desired. The plane normal of the membrane (CA₃) should therefore not intersect with the inner walls of the flow path 7.

Aerosol is generated into the mixing chamber 42 upon operation of the actuator 6 on the second side 5.2 of the membrane 5 upon inhalation of a user or during idling without a user breathing through the device. Hence, the envelope flow generated upon inhalation entrains the generated aerosol for being output through the first opening 8, being the outlet opening of the mouthpiece 27 for being administered/supplied to a user.

Upon exhalation of a user, exhaled air will enter the flow path 7 via the first opening 8 at the mouthpiece 27 and flow via the mixing chamber 42 and the open spaces provided about the circumference of the membrane 5 between the spokes 32 and the flow path openings 39 into the storage chamber 40. The exhaled air may then exit the flow path 7 via the second opening(-s) 9 and the third opening(-s) 14. Upon exhalation, that is in the second flow direction B, the check valve 15 is configured to open and let air pass through the third opening(-s) 14 and being expelled to the outside of the flow path 7 and the nebulizer housing 17. To the contrary, the check valve 15 closes the third opening(-s) 14 upon inhalation, that is in the first flow direction A. As a result, air may substantially exclusively enter the flow path 7 via the second opening(-s) 9 upon inhalation of a user and air may substantially exclusively exit the flow path 7 via the second opening(-s) 9 and the third opening(-s) 14 upon exhalation of a user.

To evaluate the quality of the inhalation therapy and to deliver a signal for controlling the actuator, it is required to observe the way the inhalation and the exhalation through the inhalation therapy device 1 is performed. In this connection a "good therapy" equals a therapy, in which a high amount of drug is delivered to the user in a short time. This can be achieved by limiting the inhalation flow and extended inhalation durations at shortened exhalation durations without overstraining the user's inhalation behaviour. In such a configuration an unpleasant feeling for the user can be avoided, which could increase the chances that the inhalation therapy is interrupted by the user.

Hitherto, the preferred embodiments of the present disclosure found an easy to manufacture and disinfect solution that achieves a beneficial short overall therapy time, in which the inhalation time can be prolonged, and the exhalation time can be kept as short as possible and without unpleasant resistances during the exhalation.

To achieve the prolonged inhalation time, it is required that the user at least inhales against a certain resistance, i.e., a certain flow resistance. The above configuration allows to achieve the desired inhalation flow resistance and, at the same time, allows to reduce the exhalation flow resistance, as the check valve 15 facilitates the expel of air via the check valve 15 and the third opening(-s) 14 of the flow path 7 upon exhalation.

In an alternative embodiment, the third opening(-s) and the check valve 15 may also be omitted. In such an embodiment, air may upon inhalation exclusively or substantially exclusively enter the flow path 7 via the second opening(-s) 9 and air may upon exhalation exclusively or substantially exclusively be expelled from the flow path 7 via the second opening(-s) 9. This is hence a scenario, in which the entire flow volume of the inhalation and the exhalation are guided through the second opening(-s) 9 of the flow path 7. The increased inhalation resistance and/or reduced exhalation resistance in this configuration is created using a fixed geometry valve functioning as the second opening.

Turning now to the controller housing 16 in more detail. The controller housing 16 accommodates the controller 18 configured to control the actuator 6 of the membrane unit 3.

A top surface/upper surface 21 of the controller housing 16, in the handle portion 12, is rounded or curved providing for a good ergonomic handling of the controller housing/handle portion 16. The top surface/upper surface 21 is located opposite to the bottom surface of the controller housing 16 defining the support surface 19. The top surface 21 defines in a side view (see figure 2) a ridge line 22. In this context, a ridge line is to be understood as a line formed along the highest points of the upper surface in side view (longitudinal cross sectional view). An angle α₆ between the ridge line 22 and the support surface 19 is, in side view (longitudinal cross sectional view), less than 20°, optionally between 5° and 15°.

At least one control button 23 and/or at least one light indicator 24 are located on the top surface 21, here centered on the ridge line 22. A user may switch the aerosol therapy device ON and OFF by means of the control button 23. The light indicator 24 may provide feedback to the user relating to the progress of the treatment.

In the present embodiment, the nebulizer housing 17 is detachable from the controller housing 16. In this context, the nebulizer housing 17 comprises a circumferential rim 43 defining a recess 44. The controller housing 16 comprises a boss 45, wherein the recess 44 and the boss 45 are engaged, when the nebulizer housing 16 of the controller housing 16 are assembled.

Further, the controller housing 16 comprises a socket 46. Upon mounting of the nebulizer housing 17 into the controller housing 16, the plug 33 of the membrane unit 3 may releasably be plugged into the socket 46, whereby the membrane unit 3 is electrically connected to the controller 18 via the socket 46.

In an example, an angle α₅ between the support surface 19 and a plugging direction of the plug 33 into the socket 46 is, in side view (longitudinal cross sectional view), more than 90° ±5°%, optionally between 100° and 160° and more optionally between 110° and 150°.

Further, a sensor 47 is accommodated in the controller housing 16. The sensor 47 is electrically connected to the controller 18. In the present example, the sensor 47 is a sensor for detecting a differential pressure between ambient pressure and a pressure in the flow path, here between the second opening(-s) at 9 and the membrane 5, that is in the present embodiment the storage chamber 40. For this purpose, the sensor 47 has a sensor port 48. The sensor port 48 is in the present embodiment located in the bottom wall 51 of the nebulizer housing 17. A tube or hose 49 connects the sensor port 48 with the sensor 47. The ambient pressure is in this configuration taken from the interior of the controller housing 16, which is not hermetically sealed so that the pressure within the controller housing 16 corresponds to ambient pressure.

Furthermore, the controller housing comprises and accommodates a battery or batteries 50. The battery or batteries may be rechargeable and/or exchangeable. The battery/-ies is/are disposed in proximity of the support surface 19 and extending with its/their length/-s along the support surface 19. In this context, along the support surface 19 does not necessarily mean that they extend parallel to the support surface 19. Yet, the longitudinal direction of the battery/-ies should be within 0° and 20° and optionally between 0° and 15° relative to the support surface 19.

Taking the aforesaid into account, the controller housing 16 may comprise the most relevant, preferably all, electronic features necessary to operate the membrane unit 3 and other features of the inhalation therapy device 1 such that the nebulizer housing 17 can be configured as a throw-away (disposable) unit or a unit, which is to be used for a certain amount of time, such as a certain number of therapies, weeks, months, half-year or maximal one year. At the same time, the controller housing 16 comprising all relevant electric and sensory elements for operating the membrane unit 3 and other features of the inhalation therapy device and monitoring the therapy process may be construed as a long-lasting unit, which does not need to be specifically disinfected before every therapy session.

The embodiment illustrated in the figures is an inhalation therapy device 1 through which a user inhales and exhales during the therapy. That is, the inhalation therapy device 1 is brought into contact with the user's mouth such that the regular inhalation and exhalation of the user is performed vie the first opening 8 through the inhalation therapy device 1. In an alternative embodiment, a mask may be attached to the first opening 8. In either case, the inhalation therapy device 1, specifically its first opening 8 shall not be removed from the user's mouth (or face if a mask is used) during the treatment. Yet, in case the patient removes the inhalation therapy device 1, specifically its first opening 8, from the user's mouth (or face if a mask is used) during the treatment, the membrane unit 3 is simply deactivated and stops aerosolizing. Hence, breaks are possible, though not intended.

How the aerosol is generated and how the actual therapy is to be performed inside the inhalation therapy device 1 will be described in the following.

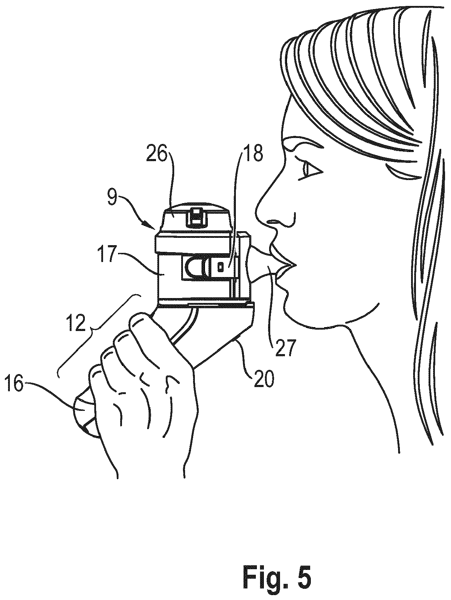

Here and in the following, it shall be understood that Figure 5 shows the orientation of the inhalation therapy device when it is held by a user in an ideal position during therapy in a standing or sitting position of the user.

In this scenario, a reservoir 2 for holding a liquid or fluid (i.e., the medicament) is provided at an upper section of the housing body 4 with the central axis CA₂ of the filling opening 25 being substantially vertical.

Additionally, it is derivable from Figure 5 that also the membrane unit 3 is oriented vertically. Thus, the center axis CA₃ of the membrane 5 is oriented horizontally. Accordingly, any liquid in the reservoir 2 is supplied to the membrane 5 by gravitational force.

As mentioned earlier, it is the first opening 8 that is to be brought into contact with the user's respiratory tract via his or her mouth to enable inhalation and exhalation through the inhalation therapy device during the aerosol therapy.

In use, when a user wants to start a therapy, the first step is to completely assemble the inhalation therapy device 1 including inserting the membrane unit 3 into the nebulizer housing 17, closing the housing 17, and attaching the nebulizer housing 17 to the controller housing 16. Moreover, the lid 26 has to be unscrewed from the reservoir 2 and the to be administered liquid has to be poured into the reservoir 2 via the filling opening 25. when the inhalation therapy device 1 is placed with its support surface 19 on a horizontal surface such as a table, a plane of the filling opening 25 will be angled towards the mouthpiece 27 relative to the horizontal surface. Yet, pouring liquid into the reservoir 2 via the filling opening 25 is still enabled without liquid flowing out of the reservoir 2. Subsequently, the lid 26 will again be screwed to the reservoir 2 to seal the liquid. In a final step, the user switches the inhalation therapy device 1 ON by a pressing the control button 23. At this stage, the user may start the treatment.

To start a treatment, the user/user will put the mouthpiece 27 in his/her mouth and start inhaling and exhaling. In this context, one breathing cycle consists of only one inhalation phase and only one subsequent exhalation phase.

When the user starts inhaling, the user will inhale against the maximum absolute value of the first flow resistance of about 160 Pa ± 100 Pa, optionally 160Pa ± 60Pa, more optionally 160Pa ± 30Pa, measured at a peak flow of a sinus-shaped breathing pattern of two seconds of inhalation time and two seconds of exhalation time, respectively, and 500 ml of tidal volume. In another embodiment, the maximum absolute value of the first flow resistance may be between 120 Pa to 500 Pa, optionally between 125 Pa to 400 Pa, even more optionally between 130 Pa to 300 Pa, and most optionally between 135 Pa to 180 Pa. In a further embodiment, the maximum absolute value of the first flow resistance upon inhalation of the user may be between 60Pa and 260Pa, optionally 100Pa and 220Pa, more optionally 130Pa and 190Pa.

In the course of the inhalation, an inhalation flow A, as described above, will be induced in the flow path 7. In the current embodiment one pressure port (or joint) of the differential pressure sensor in the controller is in fluidic communication with the flow path 7 via the sensor port 48. The other pressure port (or joint) of the differential pressure sensor in the controller is in fluidic communication with the environment such, that the differential pressure sensor 47 can measure the pressure difference between the environment (ambient pressure) and the inside of the nebulizing unit. Upon inhalation, the pressure in the flow path will be a negative pressure.

The controller 18 may, based on this output from the sensor 47, conclude on inhalation of a user. Upon conclusion on inhalation or the end of exhalation (in the case of active pre-on), the controller 18 activates the actuator 6, whereby the membrane 5 is vibrated and aerosol is generated from the liquid in the reservoir 2 on the second side 5.2 of the membrane 5. Due to the inhalation flow A, the generated aerosol will be inhaled by the user and be transported to the desired location within the respiratory tract.

At the end of the first inhalation phase, the controller 18 concludes from the pressure signal inside the flow path that the inhalation phase is close to an end, therefore deactivates the actuator 6 and, thereby, stop the aerosol production. In a particular example, the controller 18 will before the inhalation phase ends deactivate the actuator 6 and, thereby, stop the aerosol production (Pre-Off process). Accordingly, at the end of the inhalation phase, the user will no longer inhale aerosol. The timing in this regard is preferably adapted to allow that all aerosol reaches the desired location (the lung) and will not remain unused in the trachea and throat (respiratory dead volume) at time exhalation starts, maximizing the dose delivered to the user's lung. The time period during which the actuator 6 is stopped at the end of the inhalation phase may be between 100 ms and 1000 ms, optionally 300 ms to 800 ms, most optionally 500 ms before the exhalation phase starts, related to the standard sinus-shaped breathing pattern of two seconds of inhalation time and two seconds of exhalation time, respectively, and 500 ml of tidal volume. As an alternative to the time, the controller may conclude on an exhalation target volume (target respiratory dead volume) of 150ml, optionally 130ml and more optionally 100ml. Due to the Pre-Off process, all or at least most of the aerosol will reach the desired location within the respiratory tract and only a small amount of aerosol will again be exhaled in the consecutive exhalation phase.

In the consecutive exhalation phase, the user exhales producing the exhalation flow B in the flow path against a maximum absolute value of a second flow resistance of a range between 120Pa ± 75Pa, preferred 120Pa ± 45Pa, more preferred 120Pa ± 22,5Pa, measured at the peak flow of the sinus-shaped breathing pattern of two seconds of inhalation time and two seconds of exhalation time, respectively, and 500 ml of tidal volume.

When starting exhalation, the pressure within the flow path 7 again changes to overpressure and the controller 18 may, based on the output from the sensor 47, conclude on exhalation of a user. At the end of the exhalation phase, the overpressure within the flow path 7 will be reduced, approaching ambient pressure allowing the controller 18 to conclude that the exhalation phase is close to an end. In a particular example, the controller 18 will therefore before the exhalation phase ends activate the actuator 6 and, thereby, start the aerosol production (Pre-On process). The time period during which the actuator is operated during the exhalation phase may be between 50 ms and 500 ms, optionally 100 ms to 300 ms, most optionally 200 ms, related to a standard sinus-shaped breathing pattern of two seconds of inhalation time and two seconds of exhalation time, respectively, and 500 ml of tidal volume. Alternatively, time period during which the actuator is operated during the exhalation phase may be up to 500ms, up to 300ms, up to 200ms or up to 100ms.

As the aerosol production is already started during the exhalation phase and when an exhalation flow B is still present, some of the produced aerosol will be transported to a position downstream of the membrane 5 in a direction of the second opening(-s) 9 and the optional third opening(-s) 14. In order to prevent that produced aerosol during this time period is flown out of the inhalation therapy device 1, that is through the second opening(-s) 9 and the optional third opening(-s) 14, the storage chamber 40 is provided. In other words, the produced aerosol is flown during this time period into the storage chamber 40 to temporarily store the aerosol as a bolus until the next consecutive inhalation phase starts.

Subsequently, the user will start the next breathing cycle with the next inhalation phase. At the beginning of inhalation, the aerosol temporarily stored in the storage chamber 40 will again be entrained by the inhalation flow A, past the membrane unit 3 and be inhaled via the first opening 8. Upon starting with next inhalation phase, the pressure in the storage chamber 40 will again change allowing the controller 18 to conclude on the next inhalation phase.

Thus, the controller 18 will again activate the actuator 6 starting with the aerosol production. At this stage, the above-described process is repeatedly performed.

It is to be mentioned, that aerosol production may be continuous between the process Pre-On and Pre-Off (phase of operation). Yet, the power supplied to the actuator 6 (power adaption), the frequency (frequency adaption), etc. may change between consecutive phases of operation or even within one phase of operation.

If no liquid is left in the reservoir 2 or if the amount of liquid remaining in the reservoir has reached the minimum needed to produce aerosol, end of dose is reached and the process (therapy) is stopped. End of dose may be indicated to the user visually using the light indicator 24 or a light integrated into the control button 23 and/or auditory by outputting and audio signal. In this regard, end of does can be detected as described in EP 1 558 315 B1 (US 7,458,372 B2) or EP 3 082 918 B1 (US 10,744,277 B2).

Finally, the user may switch the inhalation therapy device again OFF using the controller bottom 23 to conclude the therapy. Alternatively, the device may automatically switch of after end of dose has reached and a certain amount of time has lapsed since then.

## Claims

1. Inhalation therapy device comprising:
a housing body (4),
a reservoir (2) for holding a liquid in the housing body (4),
a membrane (5) disposed in the housing body (4) and having a plurality of apertures (110), wherein the liquid is suppliable to a first side (5.1) of the membrane (5) by gravitational force;
an actuator (6) coupled to the membrane (5) for vibrating the membrane (5), whereby the liquid passes through the apertures (110) and an aerosol is generated at a second side (5.2) of the membrane (5) opposite to the first side (5.1);
a flow path (7) being defined in the housing body (4) and having a first opening (8) to the outside of the housing body (4) at one end and at least one second opening(-s) (9) to the outside of the housing body (4) at another end, so that a flow is generatable in a first flow direction (A) from the second opening(-s) (9) to the first opening (8) in the flow path (7) upon inhalation of a user at the first opening (8) for entraining and delivering the generated aerosol, and/or in a second flow direction (B) from the first opening (8) to the second opening(-s) (9) in the flow path (7) upon exhalation of a user at the first opening (8);
a sensor (47) configured to detect a flow parameter of the flow in the flow path (7),
a controller (18) configured to conclude based on the output of the sensor (47) at least on an inhalation phase and/or an exhalation phase of a user and to, during a single treatment time, repeatedly operate the actuator (6) during a period of the inhalation phase and not operate the actuator (6) during a period of the exhalation phase,
wherein an aerosol output rate of the inhalation therapy device at the first opening (8) during the single treatment time is, when using a test solution of albuterol 0,1 % (M/V) concentration in 0,9 % sodium chloride as the liquid,
a) at least 0.48 g/min, optionally at least 0.64 g/min and more optionally at least 0.8 g/min at a Median Mass Diameter of aerosol droplets between 4.5pm and 5.0pm, or
b) at least 0.42 g/min, optionally at least 0.54 g/min and more optionally at least 0.70 g/min at a Median Mass Diameter of aerosol droplets between 4.0µm and 4.5pm, or
c) at least 0.29 g/min, optionally at least 0.38 g/min and more optionally at least 0.51 g/min at a Median Mass Diameter of aerosol droplets between 3.5pm and 4.0pm, or
d) at least 0.20 g/min, optionally at least 0.29 g/min and more optionally at least 0.38 g/min at a Median Mass Diameter of aerosol droplets between 3.0µm and 3.5pm, or
e) at least 0.13 g/min, optionally at least 0.19 g/min and more optionally at least 0.26 g/min at a Median Mass Diameter of aerosol droplets between 2.5pm and 3.0µm.

2. The inhalation therapy device as defined in claim 1, wherein the controller (18) is configured to, within each breathing cycle including one inhalation phase and one exhalation phase, start operating the actuator (6) within the exhalation phase and before the inhalation phase starts.

3. The inhalation therapy device as defined in any of the preceding claims, further comprising a storage chamber (40) formed between the membrane (5) and the second opening(-s).

4. The inhalation therapy device as defined in claim 3, wherein the volume of the storage chamber (40) is between 10ml and 100ml, optionally between 20ml and 80ml, more optionally between 25ml and 75ml.

5. The inhalation therapy device as defined in any of the preceding claims, wherein the sensor (47) is configured to sense the flow parameter at the membrane (5) or between the membrane (5) and the second opening(-s).

6. The inhalation therapy device as defined in any of the preceding claims, wherein the membrane (5) is disposed in the flow path (7), whereby an envelope flow about at least part of the circumference of the membrane (5) is formed.

7. The inhalation therapy device as defined in any of the preceding claims, wherein the controller (18) is configured to, within each breathing cycle including one inhalation phase and one exhalation phase stop operating the actuator (6) within the inhalation phase and before the exhalation phase starts.

8. The inhalation therapy device as defined in any one of the preceding claims, further comprising a mixing chamber (42) formed between the membrane (5) and the first opening.

9. The inhalation therapy device as defined in claim 7, wherein the volume of the mixing chamber (42) is between 5ml and 50ml, optionally between 10ml and 40ml, more optionally between 12.5ml and 37.5ml.

10. The inhalation therapy device as defined in claim 8 or 9, wherein the mixing chamber (42) tapers towards the first opening.

11. The inhalation therapy device as defined in any of the preceding claims, wherein the flow parameter is a pressure, optionally a differential pressure and more optionally a differential pressure between the pressure in the flow path (7) and ambient pressure.

12. The inhalation therapy device as defined in any of the preceding claims, wherein the flow path (7) is configured to provide a first flow resistance upon inhalation of a user and second flow resistance upon exhalation of the user lower than the first flow resistance upon inhalation and optionally the second flow resistance upon exhalation of the user is at least 25% lower than the first flow resistance upon inhalation.

13. The inhalation therapy device as defined in claim 1, wherein the first flow resistance is between 1.25 times and 6 times, optionally 1.25 and 3 times and more optionally 1.25 times and 2.5 times larger than the second flow resistance.

14. The inhalation therapy device as defined in any of the preceding claims, wherein the apertures (110) penetrate the membrane (5) in an extension direction from the first side (5.1) to the second side (5.2), each aperture having along its extension direction (E) at the second side (5.2) a nozzle portion (112), wherein the length (L_{A}) of the nozzle portion (122) is between 5 µm and 16 pm, optionally 5 µm and 14 pm, more optionally 5µm and 12um.

15. The inhalation therapy device as defined in claim 14, wherein the apertures (110) are laser drilled formed in at least two stages, one stage forming the nozzle portion (112) and the remaining stage(-s) forming the remainder (114, 116) of the apertures (110).
